# FASCICULE DE BREVET EUROPEEN

(11) **EP 1 131 094 B1**
(45) Date de publication et mention de la délivrance du brevet: **03.11.2004**
(21) Numéro de dépôt: 99972544.3
(22) Date de dépôt: 25.11.1999
(51) Int. Cl.: A61K 39/295

(54) **VACCIN T.D.POLIO MULTIVALENT CONTRE AU MOINS LA DIPHTERIE, LA POLIOMYELITE ET LE TETANOS**
T.D.POLIO VIELWERTIGER IMPSTOFF GEGEN MINDESTENS DIPHTERIE, POLIOMYELITIS UND TETANUS
MULTIVALENT T.D. POLIO VACCINE AGAINST AT LEAST DIPHTHERIA, POLIOMYELITIS AND TETANUS

(30) Priorité: 26.11.1998 EP 98122373
(43) Date de publication de la demande: 12.09.2001
(73) Titulaire: Aventis Pasteur MSD, 69007 Lyon (FR)
(72) Inventeur: CARTIER, Jean, René, F-69005 Lyon (FR); LAROCHE, Patrick, F-78120 Rambouillet (FR)
(74) Mandataire: Schaeffer, Nathalie Christiane
(86) Numéro de dépôt international: PCT/FR1999/002913
(87) Numéro de publication internationale: WO 2000/030678

(56) Documents cités:
- WO-A-98/00167
- WO-A-98/19702
- WO-A-99/13906
- WO-A-99/48525
- SCHEIFELE D.W. ET AL.: "Can reductions in diphtheria toxoid or aluminum content reduce the reactogenicity of booster doses of DPT vaccine? " IMMUNOLOGY AND INFECTIOUS DISEASES (OXFORD), vol. 5, 1995, pages 73-77, XP000891884
- GUPTA R K ET AL: "Adjuvants for human vaccines--current status, problems and future prospects" VACCINE, vol. 13, no. 14, 1 octobre 1995 (1995-10-01), page 1263-1276 XP004057427 cité dans la demande
- GALAZKA A M ET AL: "Immunization against diphtheria with special emphasis on immunization of adults" VACCINE, vol. 14, no. 9, 1 juin 1996 (1996-06-01), page 845-857 XP004057610 cité dans la demande
- MURDIN A D ET AL: "Inactivated poliovirus vaccine: past and present experience" VACCINE, vol. 14, no. 8, 1 juin 1996 (1996-06-01), page 735-746 XP004069556 cité dans la demande
- LAROCHE P ET AL: "The immunogenicity and safety of a new combined diphtheria, tetanus and poliomyelitis booster vaccine (Td-eIPV)." INFECTION JAN.-FEB., 1999, vol. 27, no. 1, janvier 1999 (1999-01), pages 49-56, XP000891892 ISSN: 0300-8126

## Description

La présente invention concerne un nouveau vaccin multivalent contre au moins la diphtérie, la poliomyélite et le tétanos, destiné à être utilisé comme rappel vaccinal chez une personne déjà primo-vaccinée ou sensibilisée.

### État de la technique

La diphtérie est due à *Corynebacterium diphteriae.* La transmission est essentiellement directe par les voies respiratoires. Les manifestations cliniques de la forme respiratoire relèvent de deux mécanismes. La prolifération des bactéries au niveau de la porte d'entrée détermine la symptomatologie locale, habituellement pharyngée (angine pseudo-membraneuse), parfois laryngée (croup). La diffusion de l'exotoxine de certaines souches est responsable d'angine maligne et/ou de complications viscérales (polynévrite, myocardite, syndrome malin de Marfan). De nombreuses études sérologiques, réalisées récemment dans la plupart des pays développés, ont montré que, selon l'âge, le sexe et l'histoire épidémique ou vaccinale locale, 20 à 80 % des habitants ne sont plus aujourd'hui correctement immunisés ; et que les sujets de plus de 50 ans et les sujets féminins sont particulièrement vulnérables (Rappuoli *et al.,* Vaccine 11, 576-577, 1993. Simonsen *et al.,* Acta Pathol. Microbiol. Immunol. Scand, 95, 225-231, 1987. Wirz *et al.,* Vaccine, 13, 771-773, 1995). Cette vulnérabilité est révélée ces vingt dernières années par la résurgence, parfois dramatique de la diphtérie dans les pays développés (Galazka *et al.,* Eur. J. Epidemiol, 11, 107-117, 1995).

Le tétanos est dû à *Clostridium tetani.* La forme sporulée, essentiellement tellurique, introduite par effraction dans l'organisme (blessure, piqûre, brûlure ou plaie mineure), donne naissance à la forme végétative. Cette forme sécrète une endotoxine, la tétanospasmine. La lyse bactérienne provoque sa diffusion. Neurotrope, la toxine détermine la symptomatologie, c'est-à-dire un trismus inaugural et des contractures musculaires. Sans traitement, l'évolution est fatale par asphyxie ou syncope. La vaccination par anatoxine reste la seule parade. L'immunité acquise par primo-vaccination décroît avec le temps et nécessite des rappels. L'absence ou la perte avec l'âge de l'immunité a été notée dans la population américaine depuis des décennies par plusieurs enquêtes sérologiques (Hilton *et al.,* Ann. Intern. Med., 115, 32-33, 1991). En Europe, des enquêtes sérologiques ont donné des résultats équivalents (Kjeldsen *et al.,* Scand. J. Infect. Dis., 20, 177-185, 1988). Il est donc clair que de nombreux adultes, particulièrement les plus de 50 ans et les femmes, sont redevenus ou ont toujours été vulnérables au tétanos et nécessitent une vaccination par anatoxine.

La poliomyélite est due au poliovirus, appartenant au groupe des entérovirus. Il existe trois sérotypes, dénommés type 1, type 2 et type 3. La transmission est essentiellement fécale-orale, directe ou indirecte, et parfois orale-orale. Le virus est doué d'un tropisme intestinal, musculaire, méningé et nerveux. L'infection est, en général inapparente ou fruste. Cette "poliomyélite-infection" est immunisante, mais sans immunité croisée entre les types, et contagieuse pendant plusieurs semaines. Elle suscite exceptionnellement (1 ‰ à 1 % des infections selon l'âge et le type) des formes méningées ou paralytiques (paralysie flasque aiguë). Cette "poliomyélite-maladie" est toujours grave car il n'existe pas de traitement curatif étiologique. Lorsqu'elle n'est pas mortelle par asphyxie (2 à 10 % selon l'âge et le type), elle est suivie de séquelles musculaires ou ventilatoires invalidantes. Les échanges humains, toujours plus nombreux, entre les pays d'endémie et les pays indemnes de poliomyélite, exposent ces derniers aux risques d'importation et de ré-introduction de souches sauvages. Ces dernières années, en dépit d'un niveau élevé de couverture vaccinale et d'une très faible incidence globale de la maladie, des cas importés et des épidémies sont apparus en Europe, en Amérique du Nord et au Moyen-Orient (OMS, Relevé Epidémiologique Hebdomadaire, 29, 220-221, 1996). En attendant une élimination de la maladie, les nombreux échanges humains entre les pays d'endémie et les pays indemnes risquent donc de provoquer dans n'importe quelle région du monde des flambées dues à des souches sauvages importées. Afin de réduire au minimum la propagation de la maladie lors de poussées de ce type, il importe que tous les pays, y compris ceux où plus aucun cas de poliomyélite aiguë n'est signalé, maintiennent un taux élevé de couverture vaccinale dans toute leur population.

Mis au point par Ramon dans les années 1930, les vaccins diphtérique et tétanique sont à base d'anatoxines. Elles peuvent être obtenues par l'action détoxifiante du formaldéhyde sur un concentré de culture de *Corynebacterium diphteriae* ou de *Clostridium tetani,* puis purification. Leurs activités immunisantes respectives sont appréciées *in vitro* et *in vivo.* Un test de floculation mesure la quantité d'anatoxine, exprimée en unité de floculation (Lf) par dose (Lyng *et al.,* J. Biol. Stand., 15, 27-37, 1987 ; J. Lyng, Biologicals, 18, 11-17, 1990)

L'anatoxine tétanique est parfois administrée seule. Cependant, cette anatoxine est historiquement toujours combinée, au moins à l'anatoxine diphtérique. En général, la primo-vaccination tétanique et diphtérique est effectuée au cours de la première année de vie en 3 doses (OMS, WHO/EP/GEN, 95.3, 1995). Selon les pays, une dose de rappel est administrée au cours de la deuxième année et/ou entre 4 et 10 ans. Parfois, un rappel est également effectué entre 11 et 18 ans. En outre, l'OMS recommande, depuis 1987, de vacciner par anatoxine tétanique les femmes en âge de procréer dans les pays en développement, par 3 doses primo-vaccinales puis 1 dose de rappel 1 et 5 ans après.

La quantité d'anatoxine tétanique (T) par dose vaccinale varie selon les pays de 5 à 20 Lf pour une dose de 0,5 ml en primo-vaccination ou en rappel. La Pharmacopée Européenne recommande une activité d'au moins 20 UI.

La quantité d'anatoxine diphtérique (D) par dose vaccinale la plus largement et la plus anciennement administrée est celle qui existe dans les combinaisons primo-vaccinales pédiatriques : elle varie de 12 à 50 Lf pour une dose de 0,5 ml (Galazka *et al.,* Vaccine, 14, 845-857, 1996).

Pour un rappel vaccinal, la réduction de la quantité d'anatoxine diphtérique (d) s'est maintenant aussi généralisée. La quantité est généralement de l'ordre de 1/10 de la quantité pédiatrique. Elle est ainsi fixée à 2 Lf pour une dose de 0,5 ml aux US et au Canada, et est recommandée en rappel à partir de l'âge de 7 ans (Edsall *et al.,* Am. J. Hyg, 53, 283-295, 1951). En Europe, la quantité d'anatoxine et l'âge inférieur d'administration changent selon les pays. La quantité n'est pas fixée pour la raison que la seule condition imposée par la Pharmacopée Européenne porte non pas sur la quantité mais sur l'activité : elle doit être au moins de 2 UI par dose de 0,5 ml.

Pour les vaccins de rappel destinés à une population adulte, l'anatoxine diphtérique est généralement aussi combinée à l'anatoxine tétanique. Il est connu que cette combinaison réduit quelque peu la tolérance de l'anatoxine tétanique seule (Palmer *et al.,* Br. Med. J., 286, 624-626, 1983). Aux Etats-Unis, les rapports d'évènements indésirables liés aux vaccins T et Td administrés chez les sujets de plus de 7 ans ont été récemment analysés (Haber et al., ICAAC, 1996). Le vaccin Td a exposé davantage que le vaccin T à une réaction locale, une dyspnée, une perte de connaissance ou une convulsion.

Pour ce qui concerne la poliomyélite, deux vaccins ont été mis au point dans les années 1950 : le vaccin injectable (VPI), inactivé au formaldéhyde, développé par Salk (Plotkin et al., E.A. Vaccines, ed. Raven Press, 1994), et le vaccin oral (VPO), vivant atténué, développé par Sabin (Plotkin et al., *supra*). Le VPI a permis à tous les pays d'éliminer la poliomyélite, tant sauvage que postvaccinale (Murdin et al., Vaccine, 14, 735-746, 1996). Le mode de production et la composition du VPI varie selon les pays. Les trois types de poliovirus sont ainsi cultivés sur lignée cellulaire continue VERO, puis purifiés et inactivés par le formaldéhyde. Leur activité immunisante respective est appréciée *in vitro* et *in vivo.* Un ELISA mesure la teneur en antigène viral, exprimée en unités internationales : l'OMS recommande par dose de 0,5 ml 40 UI, 8 UI et 32 UI (telles que déterminées par la méthode sigmoïde} respectivement pour les antigènes D des types 1, 2 et 3.

Le VPI offre deux avantages pharmaceutiques : il est stable et ne nécessite pas de logistique particulière. Il peut être parfois combiné à d'autres antigènes.

La vaccination simultanée pendant l'enfance contre la poliomyélite, le tétanos et la diphtérie est une pratique courante depuis des années dans le monde entier, voire depuis des décennies dans la plupart des pays développés. Cette pratique a fortement contribué à la réduction considérable du nombre de cas et de morts dus à ces trois maladies. Cependant, elles n'ont pas disparu. Dans les pays développés, lorsqu'elles surviennent, elles sont d'autant plus graves qu'elles sont plus tardives. Or l'immunité acquise par vaccination s'atténue avec le temps. Puisqu'ils ne bénéficient pas d'un entretien naturel de cette immunité, les adolescents et les adultes de ces pays redeviennent vulnérables. Le maintien de la couverture vaccinale contre chacune des trois maladies tout au long de la vie est désormais une obligation épidémiologique.

Il existe donc un besoin pour un nouveau vaccin contre au moins la diphtérie, la poliomyélite et le tétanos utilisable en rappel vaccinal, qui viserait à prolonger une protection contre le tétanos, la poliomyélite et la diphtérie, conférée initialement lors d'une primo-vaccination ou une sensibilisation, et qui minimiserait sur cette population les effets indésirables induits par les vaccins existants.

Les vaccins pédiatriques injectables du genre TDPolio (T : anatoxine tétanique ; D : dose classique d'anatoxine diphtérique ; Polio : poliovirus inactivés types 1, 2 et 3), par exemple le vaccin D.T.Polio@ (PMsv S.A., France) ne peuvent satisfaire ce besoin. En effet, ces types de vaccin sont utilisés principalement en primo-vaccination pédiatrique. Ils sont dépourvus de sels d'aluminium. Ils contiennent des quantités trop importantes d'anatoxine diphtérique, de l'ordre de 100 Lf /ml, par exemple. Et ils déclenchent des réactions indésirables chez l'adulte (Björkholm *et al.,* Eur. J. Clin. Microb., 6, 637-640, 1987).

On ne peut aussi envisager de simplement modifier un vaccin TDPolio existant afin d'en minimiser les effets indésirables sur une population primo-vaccinée. En effet, une cible de choix serait par exemple d'y réduire la quantité d'anatoxine diphtérique. Dans ce cas, on n'envisagerait pas de retenir les quantités d'anatoxine utilisées classiquement dans un vaccin Td (qui est la seule référence actuelle), de l'ordre d'au moins 4 Lf par ml, par exemple, mais plutôt d'en mettre plus, probablement plus de 20 Lf/ml, par exemple. En effet, dans un vaccin Td classique l'anatoxine diphtérique est toujours adjuvantée par un sel d'aluminium, ce qui renforce son immunogénicité. Dans un vaccin TDPolio, le fait de ne pas avoir d'adjuvant implique de renforcer la dose d'anatoxine par rapport à celle utilisée dans un vaccin Td.

Pour les mêmes raisons, la simple combinaison d'un vaccin existant du genre Td injectable, par exemple les vaccins vaccinol® ou Td-Pur® (Chiron-Behring GmBH, Allemagne) ou le vaccin Diftavax® (PMsv S.A., France), avec un vaccin classique contre la poliomyélite (PMsv, France), ne pourrait, non plus, conduire à une solution satisfaisante. En effet, le choix de chaque constituant dans un tel vaccin, ainsi que leur dosage, sont déterminants pour l'obtention d'une réponse immunitaire optimale, et une minimisation des effets indésirables.

Ainsi par exemple si l'on rajoute de nouveaux antigènes (Polio) à un vaccin Td, la quantité de sels d'aluminium est alors susceptible d'être insuffisante pour jouer un rôle adjuvant optimal. L'immunogénicité d'un tel vaccin risquerait d'être ainsi réduite. Par contre, si l'on veut pallier ce problème en augmentant la quantité de sels d'aluminium, on risquerait parallèlement d'aggraver les réactions indésirables liées à ces sels (1,2 à 3 mg par ml dans les vaccins Td ; Gupta *et al.,* Vaccine, 13, 1263-1276, 1995).

De même, si l'on rajoute de nouveaux antigènes (Polio) à un vaccin Td, on diminue aussi la charge relative de chaque antigène T ou d. L'immunogénicité d'un tel vaccin risquerait d'être ainsi aussi réduite. Par contre, si l'on veut pallier ce problème en augmentant le dosage de chaque antigène, notamment celui de l'anatoxine diphtérique, on risquerait parallèlement d'aggraver les réactions indésirables liées à cette anatoxine (Björkholm *et al.,* Eur. J. Clin. Microb., 6, 637-640, 1987).

Le choix de chaque constituant d'un vaccin du type TdPolio, ainsi que leur dosage, sont donc difficiles à cerner, et ne peuvent être déduit aisément des vaccins existants.

Il n'existe pas non plus une combinaison vaccinale, appropriée pour une utilisation comme rappel vaccinal chez l'adulte ou l'adolescent, contre la diphtérie, le tétanos, la poliomyélite et la coqueluche, voire même aussi contre l'hépatite A et/ou l'hépatite B.

La présente invention vise ainsi à fournir un vaccin ayant au moins la combinaison de base TdPolio, qui, tout en se distinguant des vaccins TDPolio, Td et Polio précédents, présente une immunogénicité comparable à ceux-ci et en plus minimise leurs effets indésirables. Le vaccin selon l'invention est utilisable en vaccination de rappel.

### Résumé de l'invention

A cet effet, l'invention concerne un vaccin comprenant :
- moins de 1,2 mg par ml de sel d'aluminium, exprimé par rapport à l'atome d'Al³⁺,
- des antigènes immunogènes provenant au moins du poliovirus, de *Corynebacterium diphteriae* et de *Clostridium tetani,* et
- une quantité d'anatoxine diphtérique utilisée comme antigène immunogène de *Corynebacterium diphteriae* comprise entre 4-16 Lf par ml.

Le vaccin selon la présente invention est utilisable en primo-vaccination et comme vaccin de rappel chez une population primo-vaccinée ou sensibilisée. La demanderesse a mis en évidence que le vaccin tel que défini ci-dessus est particulièrement bien approprié pour une utilisation comme rappel vaccinal.

La demanderesse a mis en évidence de façon surprenante que le vaccin tel que défini ci-dessus, permet de minimiser les effets réactogènes et/ou allergiques induits par les antigènes constitutifs.

Selon un autre objet, la présente invention concerne une méthode de vaccination contre au moins le poliovirus, *Corynebacterium diphteriae* et *Clostridium tetani* comprenant l'administration d'un vaccin tel que défini ci-dessus.

Enfin l'invention concerne également un kit pharmaceutique comprenant au moins 2 doses injectables d'un vaccin selon la présente invention.

### Description détaillée de l'invention

Dans le contexte de la présente invention, on entend par l'expression « une population déjà primo-vaccinée ou sensibilisée », des personnes adultes, adolescentes ou juvéniles ayant déjà été vaccinées contre au moins le poliovirus, *Corynebacterium diphteriae* et/ou *Clostridium tetani,* ou des personnes ayant déjà été en contact avec l'un au moins des germes poliovirus, *Corynebacterium diphteriae,* et *Clostridium tetani;* la population préférée étant constituée par les adolescents et les adultes, et plus particulièrement les personnes âgées.

De préférence, les antigènes de *Corynebacterium diphteriae* et de *Clostridium tetani* ont pour origine leurs toxines, qui sont détoxifiées par le formaldéhyde puis purifiées. Les techniques de détoxification et de purification de ces toxines sont bien connues depuis des décennies et sont décrites par Leong *et al.,* Science, 220, 815-517, 1983; Ramon G., Ann. Inst. Pasteur, 38, 1-105, 1924; Raynaud *et al.,* Ann. Inst. Pasteur, 96 60-71, 1959 ; ou par Bizzini *et al.,* Eur. J. Biochem., 17, 100-105, 1970, par exemple. Les analogues détoxifiés pouvant être produits par génie génétique sont également compris dans la présente invention.

De même, les antigènes du poliovirus peuvent être constitués simplement par un ou plusieurs types de poliovirus inactivé (voir ci-après) et/ou par des antigènes immunogènes purifiés du poliovirus tels que ceux décrits par Delpeyroux *et al.,* 70, 1065-73, 1988 ; EP323861 (Pasteur Institut); EP86707 (Pasteur Institut); ou dans EP65924 (Pasteur Institut), par exemple.

Pour obtenir des poliovirus inactivés, on peut les cultiver sur des lignées cellulaires VERO, les purifier, puis les détoxifier avec du formaldéhyde, par exemple. Les techniques de culture et de détoxification du poliovirus sont bien connues depuis des décennies et sont décrites dans WO9800167 (Connaught); Dulbecco, Nature, 376, p.216, 1995 ; Cohen, Acta Leiden, 56, 65-83, 1987, et par Salk, Dev Biol Stand., 47, 247-55, 1981, par exemple.

De préférence, on utilise les poliovirus inactivés types 1 (souche MAHONEY), 2 (souche MEF 1) et 3 (souche Saukett).

La quantité d'anatoxine diphtérique utilisée par dose vaccinale comme antigène immunogène de *Corynebacterium diphteriae* doit être comprise entre 4 à 16 Lf par ml, de préférence 6-14 Lf, notamment de l'ordre de 10 Lfpar ml, par exemple. Cette quantité permet d'assurer une immunogénicité optimale, tout en minimisant les effets indésirables, comme les réactions réactogènes ou allergiques aux antigènes, par exemple.

De même la quantité d'anatoxine tétanique par dose vaccinale comme antigène immunogène de *Clostridum tetani* est comprise entre 6 à 30 Lf par ml, de préférence 8-20 Lf, notamment de l'ordre de 10 Lf par dose, par exemple. Cette quantité permet d'assurer une immunogénicité optimale, tout en minimisant les effets indésirables.

Pour mesurer la quantité d'anatoxine diphtérique ou tétanique (Lf), on utilise le test de floculation décrit par Lyng (J. Biol. Stand., 15, 27-37, 1987 ; ou Biologicals, 18, 11-17, 1990) ou l'OMS (Manual for the production and the control of vaccines, BLG/UNDP/77.1 et BLG/UNDP/77.2), en prenant en compte toutefois que l'on rapporte la pureté de l'anatoxine utilisée à 1500-1800 Lf par mg d'azote pour la diphtérie, ou 1000-1300 Lf par mg d'azote pour le tétanos.

A titre d'indication, dans la plupart des productions, la pureté de l'anatoxine dipthérique purifiée est généralement de l'ordre de 1700-1800 Lf par mg d'azote (source interne). De même, pour l'anatoxine tétanique, cette pureté est de l'ordre de 1200-1400 Lf (source interne).

En ce qui concerne les poliovirus inactivés utilisés comme antigènes immunogènes, une dose vaccinale selon l'invention peut comprendre 60-120 UI/ml d'antigène D du poliovirus type 1, 8-30 UI/ml d'antigène D du poliovirus type 2, et/ou 16-80 UI/ml d'antigène D du poliovirus type 3, par exemple. Les Unités Internationales sont déterminées selon la méthode Sigmoïde.

Dans un mode d'application particulier de la présente invention, le vaccin selon l'invention peut comprendre, en outre de la combinaison de base TdPolio décrite ci-dessus, 0,1 à 60 µg/ml des antigènes purifiés de *B. pertussis,* de préférence anatoxine de *B. pertussis* (PT) et F-HA; 0,1 à 40 µg /ml de l'antigène HBs de l'hépatite B ; et/ou 0,1 à 40 µg/ml de virus hépatite A inactivé ou un antigène de celui-ci, par exemple.

La toxine de *Bordetella pertussis,* y compris les analogues détoxifiés produits par génie génétique, peut être produite de différentes manières. Par exemple, on peut cultiver une souche de *B. pertussis* selon des méthodes traditionnelles (Sekura *et al.,* J.Biol. Chem., 258, 14647-14651, 1993), on peut isoler l'anatoxine en adsorbant le milieu de culture sur une colonne. Affi-Gel Blue® (Bio-Rad Lab, US), puis en l'éluant avec une solution riche en sels (par exemple 0,75 M de chlorure de magnésium), puis après avoir ôter les sels, on peut encore ensuite adsorber cet éluat sur une colonne d'affinité fetuin-Sépharose (composé de fetuin lié à du bromure de cyanogène) puis l'éluer avec une solution 4M d'un sel de magnésium. La toxine de *B. pertussis* peut être ensuite détoxifiée à l'aide du glutaraldéhyde selon une méthode modifiée de Munoz *et al.* (Infect. Immun., 33, 820-826, 1981) comme décrit dans la demande de brevet PCT/EP97/05378 (PMsv). De nombreuses autres méthodes sont encore à la disposition de l'homme du métier, comme celles de Irons *et al.* (Biochem. Biophys. Acta, 580, 175-185, 1979), ou celles décrites dans les brevets US4705686 et EP336736.

La F-HA peut être purifiée à partir du surnageant de culture essentiellement par le procédé décrit par Cowell et al (Infect. and Immun., 41, 1, 313-320,1983). Des promoteurs de croissance, tels que les béta-cyclodextrines méthylées, peuvent être utilisés pour augmenter le rendement en F-HA dans le surnageant. Le surnageant de culture est chargé sur une colonne d'hydroxylapatite. La F-HA est adsorbée sur la colonne, mais pas la PT. La colonne est très largement lavée au Triton X-100 pour supprimer l'endotoxine. La F-HA est ensuite éluée par utilisation de 0,5M NaCl dans du phosphate de sodium 0,1 M et, si nécessaire, passée dans une colonne de fétuine-Sepharose pour éliminer la PT résiduelle. Une purification supplémentaire peut comprendre le passage au travers d'une colonne de Sépharose CL-6B. Une alternative peut comprendre la purification de la F-HA par utilisation d'anticorps monoclonaux dirigés contre l'antigène, dans laquelle les anticorps sont fixés à une colonne d'affinité activée au CNBr. La F-HA peut également être purifiée par utilisation d'une chromatographie sur perlite, comme cela est décrit dans le brevet EP 336 736.

Une méthode appropriée pour la purification de la F-HA est également décrite à l'exemple 3 du brevet EP 0242 302. Dans le cadre de la présente invention, la F-HA est préparée selon cette méthode.

On peut produire une suspension de l'antigène de l'hépatite B selon la méthode décrite dans le brevet EP273 811 (Pasteur Vaccins), dans laquelle on produit des particules antigéniques de surface de l'hépatite B par expression à partir d'une culture de cellules CHO transfectées par un plasmide portant le gène HBsAg de façon à libérer les particules de surface antigéniques dans le milieu de culture. D'autres techniques sont bien connues de l'homme du métier telles que celles décrites dans EP864649, UE56711 ou IE48665, par exemple.

De même on peut produire un virus de l'hépatite A inactivé selon le protocole de Flehmig *et al.* (Viral Hepatitis and Liver Disease, 87-90, 1988 ; J. Med.Virol., 22, 7-16, 1987). D'autres techniques sont bien connues de l'homme du métier telles que celles décrites par Wang *et al.,* Vaccine, 13, 835-40, 1995 ; Shevtsova *et al.,* Zh Mikrobiol Epidemiol Immunobiol., 2, 55-90, 1995 ; Richtmann *et al.,* J Med Virol., 48, 147-50, 1996 ; EP 199480 ; IE48399 ; ou dans IE50191, par exemple.

Le vaccin selon l'invention comprend un ou plusieurs adjuvants choisis parmi les adjuvants reconnus comme tels, notamment tous les sels d'aluminium, comme les phosphates et hydroxydes d'aluminium ; le N-acétylmuramyl- L-alanyl-D-isoglutamyl-L-alanine-2-[1,2-dipalmitoyl-sn-glycéro-3-(hydroxyphospho-ryloxy)] (voir Sanchez-Pescador *et al.,* J. Immu., 141, 1720-1727, 1988) ; les molécules dérivées de *Quillaja saponaria,* comme le Stimulon® (Aquila, US) ; l'Iscoms® (CSL ltd, US) ; toutes molécules à base cholestérol et analogues, comme le DC Chol® (Targeted Genetics) ; le glycolipide Bay R1005® (Bayers, DE) ; les antigènes de *Leishmania brasiliensis* comme le LeIF (nom technique) disponible auprès de Corixa Corp. (US), les polymères de la famille de polyphosphazènes, comme l'Adjumer (nom technique) disponible auprès du «Virus Research Institute » (US), par exemple. Le vaccin selon la présente invention contient moins de 1,2 mg/ml, de préférence, 0,70 mg/ml de sel d'aluminium, exprimé par rapport à l'atome Al³⁺.

On peut remarquer que, par rapport aux vaccins TDPolio classique, comme le D.T. Polio® (PMsv, France), la présente invention propose pour la première fois l'adjonction d'un adjuvant, et notamment un sel d'aluminium, comme l'hydroxyde d'aluminium, par exemple.

Contre toute attente, le vaccin selon la présente invention peut comprendre une quantité de sel d'aluminium plus faible que celles rencontrées dans tous les vaccins Td classiques, comme le Td-Pur® ou Diftavax®, alors que l'on aurait pu estimer nécessaire d'en augmenter la charge suite à l'adjonction des poliovirus inactivés. Un vaccin selon la présente invention comprend moins de 1,2 mg/ml d'un sel d'aluminium, de préférence moins de 0,8 mg/ml. La quantité de sel d'aluminium est toujours exprimée par rapport à l'atome d'aluminium (Al³⁺); ce qui correspond à la seule méthode utilisable dans le domaine des vaccins. Toutes les quantités de sel d'aluminium auxquelles il est fait référence dans la présente demande sont donc exprimées de cette manière.

Un vaccin selon la présente invention peut comprendre d'autres constituants, comme des agents conservateurs tels que le 2-phénoxyéthanol et/ou le formaldéhyde, etc., par exemple.

Les différentes formulations retenues peuvent être l'une de celles décrites ci-dessus, par exemple, notamment celles comprenant d'autres antigènes provenant de *Bordetella pertussis,* de l'hépatite A ou de l'hépatite B.

Le vaccin selon l'invention peut se présenter sous la forme d'une suspension injectable, faiblement opalescente du fait de la présence d'un sel d'aluminium insoluble. La dose vaccinale est de préférence de l'ordre de 0,5 ml, contenue dans une seringue en verre pré-remplie. L'administration est effectuée par voie sous-cutanée profonde ou par voie intramusculaire, de préférence par voie intramusculaire, par exemple dans l'un des muscles deltoïde.

Dans un mode d'application particulier de la présente utilisation, plusieurs doses de vaccin sont destinées à être injectées séparément à un même sujet dans un intervalle de temps compris entre 10 jours à 3 mois, de sorte à favoriser une réaction immunitaire optimale, et de sorte à minimiser les effets réactogènes et/ou allergiques de ces antigènes. Pour une primo-vaccination, le vaccin est de préférence administré à raison de 3 doses, les deux premières doses étant administrées à un intervalle de 1 à 2 mois, la troisième dose étant séparée de la deuxième injection par un intervalle de 6 à 12 mois. Pour une vaccination de rappel utilisable sur une population primo-vaccinée ou sensibilisée, le vaccin selon la présente invention est administré à raison de 1 dose, ou de 2 doses séparées d'au moins 1 mois.

Le vaccin selon la présente invention peut avantageusement être utilisé dans une vaccination de rappel dans laquelle la primo-vaccination a été réalisée à l'aide d'un vaccin oral contre la poliomyélite.

On utilise, tant en primo-vaccination qu'en vaccination de rappel de préférence une dose de 0,5 ml comprenant 4 à 16 Lf/ml d'anatoxine diphtérique, de préférence 10 Lf/ml ; 6 à 30 Lf/ml d'anatoxine tétanique, de préférence 20 Lf/ml et respectivement et de préférence 40 UI. 8 UI et 32 UI (méthode sigmoïde) des antigènes D du poliovirus 1, 2 et 3. La quantité de sel d'aluminium présente étant de préférence de 0,70 mg/ml exprimée par rapport à l'atome Al³⁺.

Le vaccin selon l'invention permet de diminuer les effets indésirables induits par les vaccins TDPolio et Td existants lorsqu'ils sont injectés. On peut ainsi observer une diminution des réactions locales comme un engourdissement des membres, des sueurs, de la fièvre, des douleurs associés à des rougeurs, des nodules, indurations et/ou ecchymoses, et une diminution des dyspnées, pertes de connaissance ou convulsions, par exemple. Ces réactions peuvent être classées parmi les réactions allergiques, voire réactogènes, aux antigènes et/ou à un certains autres composés du vaccin, tel que le sel d'aluminium. La demanderesse a ainsi montré que le vaccin selon la présente invention est particulièrement approprié pour une vaccination de rappel, notamment chez les sujets adultes.

La présente invention est décrite plus en détail dans les exemples présentés ci-après. Les pourcentages sont donnés en poids sauf indication contraire. Il va de soi, toutefois, que ces exemples sont donnés à titre d'illustration de l'objet de l'invention dont ils ne constituent en aucune manière une limitation.

### Exemple 1 Immunogénicité du TdPolio avec différentes doses d'anatoxine diphtérique

On prépare de l'anatoxine diphtérique (d) en cultivant la souche IM 1514 N3S dans un milieu IMD pendant 15 à 18 h à 36°C, en centrifugeant le milieu, en le clarifiant, en le concentrant par ultrafiltration, en le détoxifiant à 37°C pendant 4 semaines en présence de 6/1000 de formaline, puis en purifiant la toxine jusqu'à atteindre une pureté de l'ordre de 1700 Lf par mg d'azote.

Parallèlement, on prépare de l'anatoxine tétanique (T) en cultivant la souche Harvard N°49205 IM 1472C dans un milieu Massachusetts à 35°C pendant 4 jours, en y ajoutant du NaCl et citrate de sodium, en centrifugeant le milieu, en le concentrant par ultrafiltration, en le détoxifiant pendant 2 semaines à 35°C en présence de 5,5/1000 de formaline et 5 g/l de bicarbonate de sodium, puis en purifiant la toxine jusqu'à atteindre une pureté de l'ordre de 1200 Lf par mg d'azote.

De même, on prépare les poliovirus inactivés types 1 (souche MAHONEY), 2 (souche MEF 1) et 3 (souche Saukett), selon la méthode Salk.

On mélange ensuite les 5 antigènes immunogènes ci-dessus avec de l'hydroxyde d'aluminium, du 2-phénoxyéthanol, du formaldéhyde, du milieu 199 de Hanks exempt de rouge de phénol et de l'eau. Pour ce faire, on stérilise le gel d'aluminium en présence d'eau, on ajuste le pH entre 5,6 et 6, on ajoute séquentiellement l'ADP (2 ou 8 Lf/dose) et l'ATP (10 Lf/dose), le milieu 199 de Hanks, on ajuste le pH à 6-6,9, on ajoute les trois types de poliovirus, puis on ajoute le 2-phénoxyéthanol et le formaldéhyde et éventuellement on ajuste le pH entre 6,8 et 7.

Le produit final de 0,5 ml servira de vaccin TdPolio, et contient au minimum 2 UI d'anatoxine diphtérique, au minimum 20 UI d'anatoxine tétanique purifiée, 40 UI, 8 UI et 32 UI d'antigène D (valeurs telles que mesurées par la méthode sigmoïde) respectivement pour les poliovirus inactivés du type 1, 2 et 3, 0,35 mg d'hydroxyde d'aluminium exprimé par rapport à l'atome d'aluminium Al³⁺, 2,5 µl de 2-phénoxyéthanol, 12,5 µg de formaldéhyde ; le reste étant constitué de milieu 199 de Hanks exempt de rouge de phénol et d'eau.

Une étude de phase I, a eu comme principal objectif de valider la tolérance clinique et biologique de la première administration d'un vaccin TdPolio adsorbé. Dans ce but, 31 adultes volontaires sains ont été recrutés. Trois lots du vaccin de 0,5 ml chacun ont été utilisés. Ils ne différaient que par la quantité d'anatoxine diphtérique purifiée décrite ci-dessus: 2Lf, 5Lf et 8 Lf par dose. Ils ont été attribués séquentiellement à raison de 10 sujets par lot. Chaque sujet a été vacciné par une dose d'un lot injectée dans un muscle deltoïde.

Aucune réaction générale n'a été rapportée dans les groupes vaccinés par les lots à 2,5 et 8 LE Au moins une réaction locale a été rapportée au cours de la première semaine chez 8 sujets du groupe vacciné par le lot à 2 Lf d'ADP, chez 6 sujets du groupe vacciné par le lot à 5 Lf d'ADP et chez 8 sujets du groupe vacciné par le lot à 8 Lf d'ADP, il s'agissait toujours de douleurs, associées à quelques rougeurs, nodules, indurations et/ou ecchymoses. Toutes les réactions ont disparu sans traitement et n'ont pas modifié la vie courante des sujets. Aucune réaction n'est survenue au-delà de la première semaine. Aucun événement indésirable grave n'a été déclaré par les investigateurs.

Cette étude a eu aussi comme objectif secondaire d'évaluer l'immunogénicité des trois premiers lots du vaccin adsorbé. Les résultats montrent que la réponse immunitaire aux cinq antigènes a été excellente pour les trois lots. En dépit de titres initiaux élevés dus au jeune âge des sujets et à des vaccinations récentes, un effet rappel a été obtenu pour chaque antigène.

### Exemple 2 Immunogénicité chez le jeune adulte

L'immunogénicité et l'innocuité d'un vaccin TdPolio a été déterminée pendant un essai clinique chez 508 jeunes adultes.

Pour chaque sujet, une dose de 0,5 ml d'un vaccin Td (Diftavax®) ou TdPolio a été injectée dans le muscle deltoïde gauche, et une dose de 0,5 ml du vaccin contre la poliomyélite (VPI®, PMsv) ou un placebo a été injecté dans le muscle deltoïde droit. Une dose de vaccin adsorbé Td contient de l'anatoxine tétanique (activité ≥20 IU), de l'anatoxine diphtérique (activité ≥2 IU) de l'hydroxyde d'aluminium (≤ 1,25 mg). Le vaccin VPI contient les antigènes D des poliovirus du type 1 (40 UI), 2 (8 UI) et 3 (32 UI). Le placebo a la même composition que le VPI à la différence près qu'il ne contient pas d'antigènes D. Le vaccin TdPolio a la même composition que celle décrite à l'exemple 1 (5 Lf d'ADP par dose).

Les anticorps anti-diphtérie, tétanos et poliomyélite sont déterminés à partir des sérums des sujets par test ELISA.

Les résultats montrent qu'avant vaccination quasiment tous les sujets étaient séropositifs en ce qui concerne l'ADP (99,2% ont un titre ≥ 0,01 UI/ml : protection possible ; et 92,6% ont un titre ≥ 0.1 UI/ml : protection assurée). Un mois après la vaccination avec le TdPolio, 99,6% des sujets avaient un titre d'anticorps ≥ 0,1 UI/ml et 82,4% un titre ≥ 1 UI/ml (protection à long terme). Au total, 17,4% des sujets ont présenté une séroconversion.

De même, la majorité des sujets étaient séropositifs en ce qui concerne l'anatoxine tétanique (99,6% ayant un titre ≥ 0.01 UI/ml : protection possible ; et 98,4% ayant un titre ≥ 0.1 UI/ml : protection assurée). Un mois après la vaccination avec le TdPolio, 100% des sujets ont un titre en anticorps ≥ 0,1 Ul/ml, et 99,2% ont un titre ≥ 1 UI/ml (protection à long terme). Au total, 25,2% des sujets ont présenté une séroconversion.

Pour ce qui concerne une protection contre la poliomyélite la majorité des sujets étaient séropositifs en ce qui concerne les trois types de poliovirus. Au total, 99,2% des sujets avaient un titre en anticorps ≥ 5 pour le type 1, 100% pour le type 2 et 97,6% pour le type 3. Un mois après la vaccination avec le TdPolio, 100% des sujets ont un titre ≥ 5 pour tous les types de poliovirus avec un titre minimum de 120 pour le type 1, 160 pour le type 2 et 80 pour le type 3. Au total, 63,1% des sujets ayant déjà un titre élevé en anticorps présentent une séroconversion.

### Lorsque l'on compare les résultats obtenus avec le TdPolio et ceux obtenus en combinant les vaccins de référence Td et VPI®, on obtient une réponse en anticorps équivalente.

### Exemple 3 Innocuité et tolérabilité chez le jeune adulte

L'inocuité et la tolérabilité d'un vaccin TdPolio ont été déterminées parmi 1742 jeunes adultes. Chacun de ces sujets a reçu une dose de 0,5 ml injecté dans le muscle deltoïde gauche du vaccin présenté à l'exemple 1 (ADP : 5 Lf par dose). Les effets ont été évalués 15 minutes après vaccination. L'effet local, régional et systémique ont été évalués pendant le mois suivant la vaccination.

L'effet immédiat commun est l'apparition d'une rougeur au point d'injection (0,34% des sujets) et une douleur (0,11%).

Les effets communs locaux pendant le mois suivant la vaccination sont les suivants pour 66% des sujets : douleur (64,41%), rougeur au point d'injection (9,13%), et nodules sous-cutanés (3,33%) Ces effets indésirables apparaissent les 3 premiers jours suivant la vaccination et durent 2 à 3 jours. 0,86% des sujets ont aussi rapporté des évènements d'oedème, d'inflammation, de migraine, d'engourdissement du bras, des contractions involontaires des muscles et une paraesthésie.

Les effets communs systémiques pendant le mois suivant la vaccination sont les suivants pour 18% des sujets : maux de tête (10,5%), nausées ou vomissements (2,75%), et malaises (2,41%). Aucun évènement d'urticaire ou de démangeaison généralisé n'a été rapporté. Ces évènements apparaissent les 3 premiers jours suivants la vaccination et durent 2 à 3 jours. Seul 0,23% des sujets ont une température dépassant ≥ 40°C durant les 3 premiers jours suivant la vaccination.

### Exemple 4 Innocuité et tolérabilité du TdPolio versus Td + Polio chez les jeunes personnes

Le vaccin TdPolio décrit à l'exemple 2 montre une excellente tolérabilité au cours de l'essai sur 508 jeunes adultes (exemple 2). Les résultats sont d'ailleurs comparables à ceux obtenus au cours de l'essai décrit à l'exemple 3.

En faisant la soustraction du pourcentage de sujets ayant eu au moins un signe local ou régional au site d'injection du placébo (14,8%) de ceux observés au site d'injection du vaccin Polio (36,5%), puis en additionnant ce pourcentage à celui observé au site d'injections du vaccin Td (66,7%), on observe une différence d'environ 8% sur l'apparition d'évenements indésirables (88,4% vs 80,5%). Le

### vaccin TdPolio est donc mieux toléré que l'administration simultanée des vaccins existants.

### Exemple 5 Immunogénicité chez les personnes au-delà de 40 ans

L'immunogénicité et l'innocuité d'un vaccin TdPolio ont été déterminées pendant un essai clinique chez 113 sujets de plus de 40 ans (40 à 78 ans). Tous les sujets avaient reçu une primo-vaccination (3 doses pendant un an) contre la diphtérie, le tétanos et la poliomyélite, la dernière vaccination remontant respectivement à 32 ans (minimum 15 ans), 28 ans (minimum 10 ans) et 28 ans (minimum 10 ans).

Les sujets ont reçu une injection d'une dose de 0,5 ml du TdPolio décrit à l'exemple 1 (ADP 5Lf par dose) dans le muscle deltoïde droit, et 28 jours plus tard une deuxième injection dans le muscle gauche. Les anticorps anti-diphtérie, tétanos et poliomyélite ont été évalués à partir des sérums des sujets par test ELISA.

Les résultats montrent que seulement 50% des sujets étaient initialement protégés contre le tétanos, mettant ainsi en évidence le déclin de l'immunité. 83% des sujets étaient initialement séropositifs contre les poliovirus types 1, 2 et 3. Au jour 28 suivant la première vaccination, la proportion de sujets protégés contre la diphtérie, le tétanos et le poliovirus types 1, 2 et 3 était montée à 80,5%, 97,3% et 100%, respectivement. Au jour 56 suivant l'administration de la seconde dose, la proportion de sujets protégés contre la diphtérie, le tétanos et le poliovirus types 1, 2 and 3 était montée à 93,7%, 100% et 100%, respectivement.

### Exemple 6 Inocuité et tolérabilité du TdPolio versus Td + Polio chez les personnes au-delà de 40 ans

L'inocuité et la tolérabilité du vaccin TdPolio décrit à l'exemple 5 ont été évaluées. Les résultats montrent un profil de tolérabilité similaire à celui obtenu avec l'association Td + Polio. Le vaccin TdPolio induit cependant moins d'événements indésirables sérieux (moins de 1,8 pour 1000 injections).

### Exemple 7 Vaccin rappel pour adultes d.T.Polio.PT.F-HA

On prépare une suspension vaccinale de 0,5 ml, avec ou sans conservateur, constituée de 5 Lf d'anatoxine diphtérique (ayant une pureté de 1700 Lf par mg d'azote), 5 Lf d'anatoxine tétanique (ayant une pureté de 1200 Lf par mg d'azote), 40 UI, 8 UI et 32 UI d'antigène D (valeurs telles que déterminées par la méthode sigmoïde) respectivement pour les poliovirus inactivés du type 1, 2 et 3 ; 6 µg/ml d'anatoxine purifiée (PT) et 6µg/ml de F-HA de *Bordetella pertussis,* 0,35 mg d'hydroxyde d'aluminium, et le reste étant constitué de milieu 199 de Hanks exempt de rouge de phénol et d'eau.

On peut aussi ajouter comme conservateur 2,5 µl de 2-phénoxyéthanol et 12,5 µg de formaldéhyde.

Les anatoxines diphtérique et tétanique, ainsi que les poliovirus inactivés ont été préparées comme décrit à l'exemple 1.

L'anatoxine de *B. pertussis* est préparée selon la méthode de Sekura *et al.* (J. Biol. Chem., 258, 14647-14651, 1993), détoxifiée selon un protocole modifié de Munoz *et al.* (Infect. Immun., 33, 820-826, 1981), puis préadsorbée sur un gel d'aluminium. La F-HA de *B. pertussis* est préparée par la méthode décrite à l'exemple 3 de EP 0242 302 puis préadsorbée sur un sel d'aluminium.

On mélange ensuite les antigènes immunogènes ci-dessus avec de l'hydroxyde d'aluminium, de l'eau, et le cas échéant avec les conservateurs. Pour ce faire, on stérilise le gel d'aluminium en présence d'eau, on ajuste le pH entre 5,6 et 6, on ajoute séquentiellement l'ADP, l'ATP et la PT et la F-HA de *B. pertussis,* on ajuste le pH à 6,8-7, on ajoute le milieu 199 de Hanks et les trois types de poliovirus, puis le cas échéant, on ajoute le 2-phénoxyéthanol et le formaldéhyde.

### Exemple 8 Vaccin rappel pour adulte d.T.Polio.PT.F-HA.HBs

On prépare une suspension vaccinale de 0,5 ml, sans conservateur, constituée de 5 Lf d'anatoxine diphtérique (ayant une pureté de 1700 Lf par mg d'azote), 5 Lf d'anatoxine tétanique (ayant une pureté de 1200 Lf par mg d'azote), 40 UI, 8 UI et 32 UI d'antigène D respectivement pour les poliovirus inactivés du type 1, 2 et 3 ; 6 µg/ml d'anatoxine purifiée (PT) de *Bordetella pertussis,* 6 µg/ml de F-HA de *B. pertussis,* 5 µg/ml d'antigène HBs de l'hépatite B, 0,35 mg d'hydroxyde d'aluminium, et le reste étant constitué de milieu 199 de Hanks exempt de rouge de phénol et d'eau.

Les anatoxines diphtérique et tétanique, ainsi que les poliovirus inactivés ont été préparés comme décrit à l'exemple 1. La PT et la F-HA *B. pertussis* ont été préparées comme décrit à l'exemple 7.

L'antigène HBs a été préparé selon la méthode décrite dans le brevet EP 273 811 (Pasteur Vaccins). Il est stabilisé par une préadsorbtion sur un gel d'aluminium.

On mélange ensuite les antigènes immunogènes ci-dessus avec de l'hydroxyde d'aluminium et de l'eau. Pour ce faire, on stérilise le gel d'aluminium en présence d'eau, on ajuste le pH entre 5,6 et 6, on ajoute séquentiellement l'ADP, l'ATP et PT et la F-HA de *B. pertussis,* on ajuste le pH à 6,8 - 7, on ajoute le milieu 199 de Hanks et les trois types de poliovirus, on ajuste le pH à 6,8 si nécessaire, et on ajoute l'HBs.

### Exemple 9 Vaccin rappel pour adultes d.T.Polio.PT.F-HA.HBs.HA

On prépare une suspension vaccinale de 0,5 ml, sans conservateur, constitué de 5 Lf d'anatoxine diphtérique (ayant une pureté de 1700 Lf par mg d'azote), 5 Lf d'anatoxine tétanique (ayant une pureté de 1200 Lf par mg d'azote), 40 UI, 8 UI et 32 UI d'antigène D respectivement pour les poliovirus inactivés du type 1, 2 et 3 ; 6 µg/ml d'anatoxine purifiée (PT) de *Bordetella pertussis,* 6 µg/ml de F-HA de *B. pertussis,* 5 µg/ml d'antigène HBs de l'hépatite B, 5 µg/ml de virus de l'hépatite A inactivé, 0,35 mg d'hydroxyde d'aluminium, et le reste étant constitué de milieu 199 de Hanks exempt de rouge de phénol et d'eau.

Les anatoxines diphtérique et tétanique, ainsi que les poliovirus inactivés ont été préparés comme décrit à l'exemple 1. La PT et la F-HA *B. pertussis* ont été préparées comme décrit à l'exemple 7. L'antigène HBs a été préparé comme décrit à l'exemple 8.

Le virus inactivé de l'hépatite A est préparé selon la méthode de Flehmig *et al. (supra).*

On mélange ensuite les antigènes immunogènes ci-dessus avec de l'hydroxyde d'aluminium, les conservateurs et de l'eau. Pour ce faire, on stérilise le gel d'aluminium en présence d'eau, on ajuste le pH entre 5,6 et 6, on ajoute séquentiellement l'ADP, l'ATP et la PT et la F-HA de *B. pertussis,* on ajuste le pH à 6,8 - 7, on ajoute le milieu 199 de Hanks et les trois types de poliovirus, on ajuste le pH à 6,8 si nécessaire, puis on ajoute séquentiellement l'HBs et le virus de l'hépatite A inactivé.

## Revendications

1. Un vaccin de rappel comprenant :
- moins de 1,2 mg par ml de sel d'aluminium, exprimé par rapport à l'atome Al³⁺,
- des antigènes immunogènes provenant au moins du poliovirus, de *Corynebacterium diphteriae* et de *Clostridium tetani,* et
- une quantité d'anatoxine diphtérique utilisée comme antigène immunogène de *Corynebacterium diphteriae* comprise entre 4-16 Lf par ml.

2. Un vaccin de rappel selon la revendication 1, **caractérisé en ce que** la quantité d'anatoxine diphtérique est de l'ordre de 10 Lf par ml.

3. Un vaccin de rappel selon la revendication 1 ou 2 dans lequel la quantité d'anatoxine tétanique est de l'ordre de 20 Lf par ml.

4. Un vaccin de rappel selon l'une quelconque des revendications 1 à 3 comprenant en outre au moins un antigène choisi parmi les antigènes de *Bordetella pertussis,* de l'hépatite A et de l'hépatite B.

5. Un vaccin selon l'une quelconque des revendications 1 à 4 pour minimiser les effets réactogènes et/ou allergiques induits par ces antigènes et/ou les sels d'aluminium lors d'une vaccination de rappel.

6. L'utilisation d'un vaccin selon l'une quelconque des revendications 1 à 5 pour la fabrication d'un médicament pour la protection d'un individu primo-vacciné ou sensibilisé contre au moins le poliovirus, *Corynebacterium diphteriae* et *Clostridium tetani.*

7. Utilisation selon la revendication 6, pour la préparation d'au moins deux doses de vaccin destinées à être injectées séparément à un même sujet adulte dans un intervalle de temps compris entre 10 jours à 3 mois, ledit vaccin étant destiné à minimiser les effets réactogènes et/ou allergiques induits par ces antigènes.

8. Kit pharmaceutique comprenant au moins 2 doses injectables d'un vaccin selon l'une des revendications 1 à 5.

## Claims

1. Booster vaccine comprising:
- less than 1.2 mg per ml of aluminium salt, expressed with respect to the Al³⁺ atom,
- immunogenic antigens originating at least from the poliovirus, from *Corynebacterium diphtheriae* and from *Clostridium tetani,* and
- an amount of diphtheria toxoid used as an immunogenic antigen of *Corynebacterium diphtheriae* of between 4-16 Lf per ml.

2. Booster vaccine according to Claim 1, **characterized in that** the amount of diphtheria toxoid is about 10 Lf per ml.

3. Booster vaccine according to Claim 1 or 2, in which the amount of tetanus toxoid is about 20 Lf per ml.

4. Booster vaccine according to any one of Claims 1 to 3, also comprising at least one antigen chosen from the *Bordetella pertussis,* hepatitis A and hepatitis B antigens.

5. Vaccine according to any one of Claims 1 to 4, for minimizing the reactogenic and/or allergic effects induced by these antigens and/or the aluminium salts during booster immunization.

6. Use of a vaccine according to any one of Claims 1 to 5, for manufacturing a medicinal product for protecting a primary immunized or sensitized individual against at least the poliovirus, *Corynebacterium diphtheriae* and *Clostridium tetani.*

7. Use according to Claim 6, for preparing at least two doses of vaccine intended to be injected separately into the same adult individual in a period of time of between 10 days and 3 months, said vaccine being intended to minimize the reactogenic and/or allergic effects induced by these antigens.

8. Pharmaceutical kit comprising at least 2 injectable doses of a vaccine according to one of Claims 1 to 5.

## Patentansprüche

1. Ein Auffrischimpfstoff, umfassend:
- weniger als 1,2 mg pro ml Aluminiumsalz, ausgedrückt in Bezug auf das Al³⁺-Atom,
- immunogene Antigene, die wenigstens vom Poliovirus, von *Corynebacterium diphtheriae* und von *Clostridium tetani* stammen, und
- eine Menge an Dtphtherie-Anatoxin, das als immunogenes Antigen von *Corynebacterium diphtheriae* verwendet wird, zwischen 4-16 Lf pro ml.

2. Ein Auffrischimpfstoff gemäß Anspruch 1, **dadurch gekennzeichnet, dass** die Menge an Diphtherie-Anatoxin in der Größenordnung von 10 Lf pro ml liegt.

3. Ein Auffrischimpfstoff gemäß Anspruch 1 oder 2, worin die Menge an Tetanus-Anatoxin in der Größenordnung von 20 Lf pro ml liegt.

4. Ein Auffrischimpfstoff gemäß einem der Ansprüche 1 bis 3, umfassend außerdem wenigstens ein Antigen, das ausgewählt ist unter den Antigenen von *Bordetella pertussis,* Hepatitis A und Hepatitis B.

5. Ein Impfstoff gemäß einem der Ansprüche 1 bis 4 zum Minimieren der reaktogenen und/oder allergischen Wirkungen, die von diesen Antigenen und/oder den Aluminiumsalzen bei einer Auffrischimpfung ausgelöst werden.

6. Verwendung eines Impfstoffs gemäß einem der Ansprüche 1 bis 5 für die Herstellung eines Medikaments für den Schutz eines erstgeimpften oder sensibilisierten Menschen gegen wenigstens das Poliovirus, *Corynebacterium diphtheriae* und *Clostridium tetani.*

7. Verwendung gemäß Anspruch 6 für die Herstellung von wenigstens zwei Impfstoffdosen, die dazu bestimmt sind, dem gleichen Erwachsenen in einem Zeitintervall zwischen 10 Tagen bis 3 Monaten getrennt voneinander injiziert zu werden, wobei besagter Impfstoff dazu bestimmt ist, die reaktogenen und/oder allergischen Wirkungen, die von diesen Antigenen ausgelöst werden, zu minimieren.

8. Pharmazeutisches Kit, enthaltend wenigstens 2 injizierbare Dosen eines Impfstoffs gemäß einem der Ansprüche 1 bis 5.
